# EUROPEAN PATENT APPLICATION

(11) **EP 4 578 875 A1**
(43) Date of publication of application: **02.07.2025**
(21) Application number: 23856525.3
(22) Date of filing: 16.08.2023
(51) Int. Cl.: C07K 16/28, A61K 39/395, C12N 5/10, C12N 15/13, C12N 15/63, A61P 35/00

(54) **ANTI-HER2/CD3 BISPECIFIC ANTIBODY SPECIFICALLY ACTIVATED IN TUMOR MICROENVIRONMENT**

(30) Priority: 22.08.2022 CN 202211006978
(71) Applicant: Ying Mai de Medical Technology (Guangdong) Co., Ltd., Guangzhou, Guangdong 510700 (CN)
(72) Inventor: DING, Chengli, Guangzhou, Guangdong 510700 (CN); LIU, Yuan, Guangzhou, Guangdong 510700 (CN); LIU, Chen, Guangzhou, Guangdong 510700 (CN)
(74) Representative: Mewburn Ellis LLP
(86) International application number: PCT/CN2023/113380
(87) International publication number: WO 2024/041435

(57) **Abstract**

The present invention relates to an anti-Her2/CD3 bispecific antibody specifically activated in a tumor microenvironment. Specifically, provided is a bispecific antibody capable of being chemically conjugated to a substrate that is highly expressed in a tumor environment and can be recognized and cut, as well as a masking moiety, in a chemical coupling manner. The antibody activity of the bispecific antibody of the present invention in normal tissues is suppressed, but is activated in a tumor environment. **In** addition, the bispecific antibody of the present invention also has significantly reduced toxicity.

## Description

### Technical field

The present invention relates to the field of antibody medicine. Specifically, it relates to an anti-Her2/CD3 bispecific antibody specifically activated in a tumor microenvironment.

### Background

Multispecific antibody refer to a special antibody that can bind to two different antigens or different epitopes of one antigen, which has become a research hotspot in the field of antibody engineering and has broad application prospects in fields such as tumor immunotherapy and autoimmune diseases.

In 2014, Blinatumomab, a multispecific antibody developed by Amgen, was approved by the FDA for the treatment of acute lymphoblastic leukemia. In November 2017, Emicizumab developed by Roche Pharmaceuticals was approved by the FDA for the treatment of hemophilia A. With the successful clinical application and approval for marketing of multispecific antibodies, they have become a new hotspot in drug research. However, there are still some urgent issues that need to be addressed for multispecific antibody, including adverse reactions such as cytokine storms and neurotoxicity, as well as production process problems.

Therefore, there is still a need to provide multispecific antibodies with high safety, strong specificity, and high anti-tumor activity in this field.

### Summary of the invention

The purpose of the present invention is to provide an anti-Her2/CD3 bispecific antibody specifically activated in a tumor microenvironment.

The purpose of the present invention is also to provide a method for the construction of an activatable multispecific antibody, and uses thereof.

In the first aspect of the present invention, it provides a mutant CD3 antibody, wherein one or more mutations are introduced in the complementary determining regions (CDR regions) of the heavy chain variable region and/or light chain variable region of the mutant CD3 antibody relative to a wild-type CD3 antibody sequence, wherein the mutation is selected from the group consisting of:
a mutation where one or more amino acids are replaced with cysteine (Cys) at the position of M34, S53, Y61, D68, F104, W111, or F112 in the heavy chain variable region; and/or at the position of N54, K55, R56, W93, Y94 or N96 in the light chain variable region;
wherein the affinity of the wild-type CD3 antibody for binding to CD3 is E0, the affinity of the mutant CD3 antibody for binding to CD3 is E1, and E1/E0 ≤ 5.5 (preferably E1/E0 ≤ 3, preferably ≤ 1.5, more preferably E1/E0 ≤ 1);
wherein the heavy chain variable region of the wild-type CD3 antibody comprises the following three complementary determining regions (CDRs):
   VH-CDR1 as shown in SEQ ID NO.1,
   VH-CDR2 as shown in SEQ ID NO.2, and
   VH-CDR3 as shown in SEQ ID NO.3; and
   the light chain variable region of the wild-type CD3 antibody comprises the following three complementary determining regions (CDRs):
      VL-CDR1 as shown in SEQ ID NO.5,
      VL-CDR2 as shown in SEQ ID NO.6, and
      VL-CDR3 as shown in SEQ ID NO.7.

In another preferred embodiment, the wild-type CD3 antibody includes: a single chain antibody, a double chain antibody, a monoclonal antibody, a chimeric antibody (such as human mouse chimeric antibody), a mouse derived antibody, or a humanized antibody.

In another preferred embodiment, the affinity for binding to CD3 is the EC50 value for binding to CD3.

In another preferred embodiment, the ratio of the affinity of the mutant CD3 antibody for binding CD3 (E1) to the affinity of the corresponding wild-type CD3 antibody for binding CD3 (E0) (E1/E0) is 0.4-5.5, preferably 0.5-2, preferably 0.7-1.5, and more preferably 0.8-1.2.

In another preferred embodiment, the wild-type CD3 antibody is a CD3 mouse derived antibody or a CD3 humanized antibody.

In another preferred embodiment, the wild-type CD3 antibody comprises a heavy chain variable region as shown in SEQ ID NO. 10, and a light chain variable region as shown in SEQ ID NO. 11.

In another preferred embodiment, the CD3 humanized antibody comprises a heavy chain variable region as shown in SEQ ID NO. 4 and a light chain variable region as shown in SEQ ID NO. 8.

In another preferred embodiment, the mutant CD3 antibody comprises one or more mutations in the complementarity determining regions (CDRs) of the heavy chain variable region and/or light chain variable region relative to the wild-type CD3 antibody; preferably, 1, 2, 3, 4, or 5 amino-acid mutations.

In another preferred embodiment, the complementary determining region (CDR) of the heavy chain variable region of the mutant CD3 antibody comprises a mutation in which one or more amino acids are replaced with cysteine (Cys) relative to the wild-type CD3 antibody; and/or the complementary determining region (CDR) of the light chain variable region of the mutant CD3 antibody comprises a mutation in which one or more amino acids are replaced with cysteine (Cys) relative to the wild-type CD3 antibody.

In another preferred embodiment, the mutant CD3 antibody comprises a mutation selected from the following group in the complementary determining regions of the heavy chain variable region corresponding to the wild-type CD3 antibody: M34C, S53C, Y61C, D68C, F104C, W111C, F112C, and a combination thereof, preferably a mutation of Y61C; and/or
comprises a mutation selected from the following group in the complementarity determining regions of the light chain variable region corresponding to the wild-type CD3 antibody: Y34C, N54C, K55C, R56C, W93C, Y94C, N96C, and a combination thereof, preferably a mutation of Y34C.

In another preferred embodiment, all position numbering of amino acids mentioned for antibody amino acid residues are based on Kabat numbering.

In another preferred embodiment, no interchain disulfide bonds or intrachain disulfide bonds are formed within the complementary determining regions (CDRs) of the variable region of the antibody.

In another preferred embodiment, the heavy chain variable region of the mutant CD3 antibody comprises the following three complementary determining regions (CDRs):
VH-CDR1 as shown in SEQ ID NO.1,
VH-CDR2 as shown in SEQ ID NO.9, and
VH-CDR3 as shown in SEQ ID NO.3; and
the light chain variable region comprises the following three complementary determining regions (CDR):
   VL-CDR1 as shown in SEQ ID NO.5,
   VL-CDR2 as shown in SEQ ID NO.6, and
   VL-CDR3 as shown in SEQ ID NO.7.

In another preferred embodiment, the mutant CD3 antibody includes: (i) Fab fragment; (ii) F(ab')₂ fragment; (iii) Fd fragment; (iv) Fv fragment; (v) single-stranded Fv(scFv) molecule; or (vi) dAb fragment.

In another preferred embodiment, the mutant CD3 antibody is a bispecific, trispecific, or multispecific antibody.

In another preferred embodiment, the mutant CD3 antibody includes a modified mutant CD3 antibody.

In another preferred embodiment, the modified mutant CD3 antibody is a tumor micro-environment specifically activated antibody (TMEAbody), i.e., a masked antibody.

In another preferred embodiment, the modified mutant CD3 antibody comprises, but is not limited to, linking a blocker to a cysteine at the mutation site of the mutant CD3 antibody via a cleavable linker peptide, wherein the mutation site is located in the complementary determining region (CDR region) of the heavy chain variable region and/or light chain variable region of the mutant CD3 antibody.

In another preferred embodiment, the mutant CD3 antibody comprises a modified mutant CD3 antibody having the following structure:

Ab-(P-R1)n

wherein,
Ab is a mutant CD3 antibody;
R1 is each independently a blocker;
P is a cleavable linker peptide;
n is a positive integer from 1 to 5;
"-" is a chemical bond or connector or linker.

In another preferred embodiment, the blocker refers to a group that prevents the Ab from binding to its ligand or receptor.

In another preferred embodiment, the blocker is selected from groups comprising a pegylated C₁₋₅-alkylcarbonyl group: 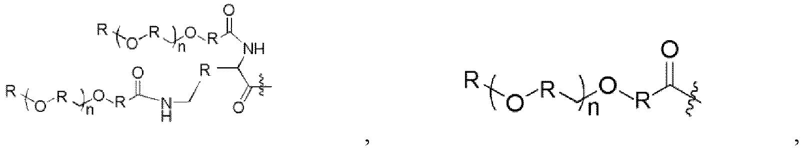 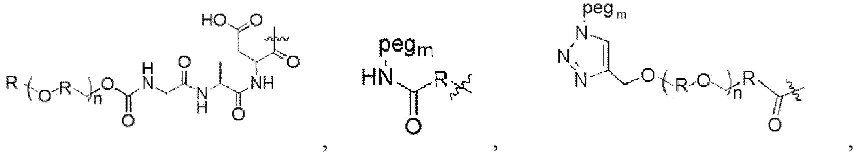 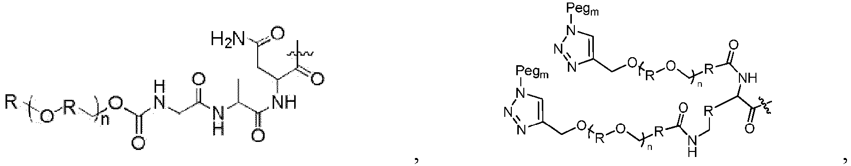 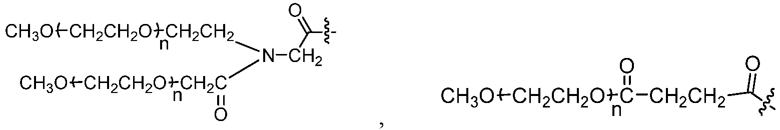 and 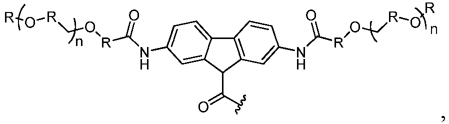 wherein, each R is independently a C1-4 alkyl group; each n is independently an integer in the range of 1 to 30000, such as 1 to 15000, 1 to 5000, 1 to 2000, 1 to 300, 1 to 150, 1 to 50, 1 to 20, or 3 to 12; polyethylene glycol or Pegₘ represents polyethylene glycol with a molecular weight of 44 to 132000, such as polyethylene glycol with a molecular weight of 1000 to 50000 or 10000 to 30000; m represents the molecular weight of polyethylene glycol; the wavy line represents the position connected to R2.

In another preferred embodiment, the blocker is selected from the group consisting of: PEG5K, PEG10K, PEG20K, or PEG40K.

In another preferred embodiment, R1 is 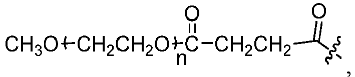 wherein, n is independently an integer from 1 to 2000, 1 to 300, 1 to 150, 1 to 50, or 1 to 20.

In another preferred embodiment, P includes the following structure:
R2-R3-R4, wherein,
R2 is absent, or a cleavable connecting arm that can be activated by one or more proteolytic enzymes, or a chemical bond that can be activated by acid in a pathological microenvironment;
R3 is absent, or a connecting arm that automatically detaches after R2 is cleaved or activated by acid in a pathological microenvironment; and when R2 is absent, R3 is a chemical bond activated by acid in a pathological microenvironment;
R4 is a group that can covalently bind to the sulfur atom in the cysteine residue comprised in the mutant CD3 antibody.

In another preferred embodiment, R1 is connected to R2 in P through a chemical bond.

In another preferred embodiment, R2 is a peptide that can be activated or cleaved by one or more proteolytic enzymes, proteases, or peptidases, wherein the protease is selected from the group consisting of: asparagine endopeptidase, granzyme, cathepsin B, cathepsin C, cathepsin D, cathepsin E, cathepsin K, cathepsin L, kallikrein, hK1, hK10, hK15, plasmin, collagenase, type IV collagenase, astrotactin, Xa factor, chymotrypsin-like protease, trypsin-like protease, elastase-like protease, subtilisin-like protease, actinidin (kiwi protease), bromelain, calpain, caspase, caspase-3, Mirl-CP, papain, HIV-1 protease, HSV protease, CMV protease, rennet, peptidase, interstitial protease, plasma protease, naprosin (pitcher plant protease), metallopeptidase, metalloendopeptidase, matrix metalloproteinase (MMP), MMP1, MMP2, MMP3, MMP8, MMP9, MMP10, MMP11, MMP12, MMP13, MMP14, ADAM10, ADAM12, urokinase plasminogen activator (uPA), enterokinase, prostate-specific antigen (PSA, hK3), interleukin-113 converting enzyme, thrombin, FAP (FAP-a), transmembrane peptidase (meprin), dipeptidyl peptidase, and dipeptidyl peptidase-4 (DPPIV/CD26), preferably asparagine endopeptidase.

In another preferred embodiment, R2 is a linker peptide cleavable under an acidic condition in the pathological microenvironment; preferably, R2 is a tripeptide, wherein the first amino acid residue of the tripeptide connected to R1 is selected from Ala, Thr, Val, and Ile, the second amino acid residue in the middle is selected from Ala, Thr, Val, and Asn, and the third amino acid residue connected to Ab is selected from Asn and Asp; wherein R2 is connected to R1 through the amino group of the first amino acid residue via amide, ester, carbamate, urea, or hydrazone bonds, and is connected to R3 through the carboxyl group of the third amino acid residue via amide, ester, carbamate, urea, or hydrazone bonds; preferably, the tripeptide is selected from Ala-Ala-Asn and Ala-Ala-Asp.

In another preferred embodiment, Ala-Ala-Asn can be recognized and cleaved by asparagine endopeptidase, and the Ala-Ala-Asp can be recognized and cleaved by granzyme.

In another preferred embodiment, R2 is a linker peptide cleavable under an acidic condition in the pathological microenvironment and is selected from amide, ester, carbamate, urea, and hydrazone bonds; preferably, wherein the structure of R1-R2-R3-R4 is represented by the following formulae: 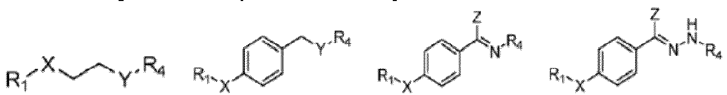 wherein X and Y are each independently NR' or O, Z is H or C1-10 alkyl, and R' is H or C1-4 alkyl; and R3 is connected to R1 and R4 through X and Y via amide, ester, carbamate, urea, or hydrazone bonds, respectively.

In another preferred embodiment, R4 is

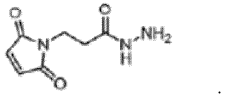

In another preferred embodiment, the structure of P-R1 is as follows, 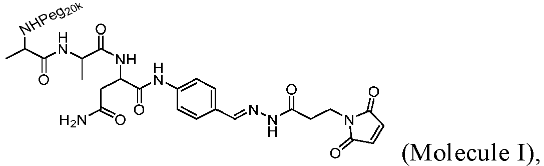 wherein, R1 is PEG20K, the structural formula of which is:

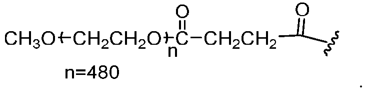

In another preferred embodiment, the modified mutant CD3 antibody is an activatable antibody.

In another preferred embodiment, the protease is produced by a tumor close to cells expressing CD3 in the tissue and/or by a tumor co-localized with CD3 in the tissue, and the protease cleaves P in the activable antibody when the activable antibody is exposed to the protease.

In another preferred embodiment, R4 in P is linked to a thiol group of the mutant CD3 antibody (Ab).

In another preferred embodiment, R4 in P is site-specifically connected to an amino acid site of the mutant CD3 antibody selected from the group consisting of: a position corresponding to M34, S53, Y61, D68, F104, W111, or F112 of the complementary determining region of the heavy chain variable region of the wild-type CD3 antibody; and/or a position corresponding to Y34, N54, K55, R56, W93, Y94, or N96 of the complementarity determining region of the light chain variable region of the wild-type CD3 antibody.

In another preferred embodiment, R4 in P is site-specifically connected to an amino acid site of the mutant CD3 antibody selected from the group consisting of: M34, S53, Y61, D68, F104, W111, or F112 of the complementary determining region of the heavy chain variable region corresponding to the wild-type CD3 antibody; and/or Y34, N54, K55, R56, W93, Y94, or N96 of the complementarity determining region of the light chain variable region corresponding to the wild-type CD3 antibody. In another preferred embodiment, R4 in P is site-specifically linked to an amino acid site of the mutant CD3 antibody selected from the group consisting of: a position corresponding to Y61 of the complementary determining region of the heavy chain variable region of the wild-type CD3 antibody; and/or a position corresponding to Y34 of the complementarity determining region of the light chain variable region of the wild-type CD3 antibody.

In another preferred embodiment, R1 has a dissociation constant selected from the following for binding to Ab:
greater than the dissociation constant between Ab and CD3; equal to the dissociation constant between Ab and CD3; and smaller than the dissociation constant between Ab and CD3.

In another preferred embodiment, when the modified mutant CD3 antibody is in the cleaved state, R1 does not interfere with the binding of Ab to CD3 or compete with Ab for CD3 binding.

In another preferred embodiment, R1 is located in the activable antibody such that when the activable antibody is in the uncleaved state, R1 interferes with the specific binding of Ab to CD3, and when the activable antibody is in the cleaved state, R1 does not interfere with the specific binding of Ab to CD3 or compete with Ab for specific binding to CD3.

In another preferred embodiment, the ratio of the affinity of the modified mutant CD3 antibody for binding to CD3 (E2) to the affinity of the corresponding wild-type CD3 antibody for binding to CD3 (E2) (E2/E0) is 1-1000, preferably 2-800, preferably 5-600, and more preferably 10-500.

In the second aspect of the present invention, it provides a multispecific antibody which comprises: the mutant CD3 antibody or a modified mutant CD3 antibody of the first aspect of the present invention.

In another preferred embodiment, the multispecific antibody further comprises one or more antigen binding regions targeting a target selected from the group consisting of: Her2, DLL3, CLDN6, EGFR, TGFβ, BCMA, B7H6, GUCY2C, CD38, CD123, CD19, CD20, CD22, B7-H3, GPC3, CD73, PMSA, CD28, 4-1BB, OX40, CD40, CD27, CTLA4, PD1, PDL1, BCMA, GLP-1, Trop2, TIGIT, LAG-3, FGL1, TLR7.

In another preferred embodiment, the antigen-binding region is an antibody or an antibody fragment, and the antibody fragment includes: (i) Fab fragment; (ii) F(ab')₂ fragment; (iii) Fd fragment; (iv) Fv fragment; (v) single-stranded Fv(scFv) molecule; (vi) dAb fragment.

In another preferred embodiment, the multispecific antibody includes a bispecific antibody or a trispecific antibody.

In another preferred embodiment, the multispecific antibody comprises a second antigen binding region targeting a target selected from the group consisting of: Her2, DLL3, CLDN6.

In another preferred embodiment, the second antigen binding region is a scFv fragment targeting Her2.

In another preferred embodiment, the multispecific antibody further comprises an Fc segment of the antibody.

In another preferred embodiment, the Fc segment comprises a Knobs-in-holes (KIH) structure.

In another preferred embodiment, the Fc segment is a human IgG Fc segment.

In another preferred embodiment, the mutant CD3 antibody of claim 1 or the modified mutant CD3 antibody of claim 2 is a Fab fragment of the CD3 antibody.

In another preferred embodiment, the heavy chain variable region of the Fab fragment of the CD3 antibody comprises the following three complementary determining regions (CDRs):
VH-CDR1 as shown in SEQ ID NO.1,
VH-CDR2 as shown in SEQ ID NO.9, and
VH-CDR3 as shown in SEQ ID NO.3; and
the light chain variable region comprises the following three complementary determining regions (CDRs):
   VL-CDR1 as shown in SEQ ID NO.5,
   VL-CDR2 as shown in SEQ ID NO.6, and
   VL-CDR3 as shown in SEQ ID NO.7.

In another preferred embodiment, the sequence of the scFv fragment targeting Her2 is as shown in SEQ ID NO. 12.

In another preferred embodiment, the multispecific antibody is a bispecific antibody, which comprises:
a first combination element D 1; and
a second combination element D2;
wherein, D1 is the mutant CD3 antibody or a derivative antibody (modified mutant CD3 antibody) thereof of the first aspect of the present invention; and
D2 specifically binds to a target molecular protein selected from the group consisting of: Her2, DLL3, and CLDN6.

In another preferred embodiment, D1 and D2 are connected by a linker peptide.

In another preferred embodiment, the linker peptide comprises an antibody constant region.

In another preferred embodiment, the antibody constant region comprises a heavy chain constant region, a light chain constant region, or an Fc segment.

In another preferred embodiment, the Fc segment comprises a Knobs-in-holes (KIH) structure.

In another preferred embodiment, the CD3 protein is a CD3 ε chain (CD3 ε).

In another preferred embodiment, D1 is a mutant CD3 antibody or a derivative antibody (modified mutant CD3 antibody) thereof.

In another preferred embodiment, the modified mutant CD3 antibody is as described in the first aspect of the invention.

In another preferred embodiment, the complementary determining region (CDR) of the heavy chain variable region of the mutant CD3 antibody comprises a mutation in which one or more amino acids are replaced with cysteine (Cys) relative to the wild-type CD3 antibody; and/or the complementary determining region (CDR) of the light chain variable region of the mutant CD3 antibody comprises a mutation in which one or more amino acids are replaced with cysteine (Cys) relative to the wild-type CD3 antibody.

In another preferred embodiment, the mutant CD3 antibody comprises an amino acid replaced with cysteine (Cys) at the position corresponding to the Y61 position of the complementary determining region of the heavy chain variable region of the wild-type CD3 antibody; and/or the Y34 position of the light chain variable region of the wild-type CD3 antibody.

In another preferred embodiment, D1 is the Fab segment of a mutant CD3 antibody.

In another preferred embodiment, D2 is an antibody or an antigen binding fragment thereof targeting a target molecular protein selected from the group consisting of: HER2, DLL3,and CLDN6, preferably HER2.

In another preferred embodiment, the structure of the antigen binding fragment is selected from the group consisting of: (i) Fab fragment; (ii) F(ab')₂ fragment; (iii) Fd fragment; (iv) Fv fragment; (v) single chain Fv (scFv) molecule; or (vi) dAb fragment.

In another preferred embodiment, D2 is a scFv of an antibody targeting HER2.

In the third aspect of the present invention, it provides a recombinant protein, wherein the recombinant protein comprises:
(1) the mutant CD3 antibody or the modified mutant CD3 antibody of the first aspect of the present invention, or the multispecific antibody of the second aspect of the present invention;
(2) an optional polypeptide molecule or fragment with therapeutic function; and/or
(3) an optional protein functional domain that assists enhancing molecular physicochemical properties and pharmaceutical properties.

In another preferred embodiment, the recombinant protein also comprises: (iv) an optional tag sequence that assists expression and/or purification.

In another preferred embodiment, the tag sequence is selected from the group consisting of: 6His tag, GGGS sequence, FLAG tag.

In another preferred embodiment, the recombinant protein is a monomer, dimer, or multimer.

In another preferred embodiment, the polypeptide molecule or fragment with therapeutic function includes but is not limited to: a peptide molecule or fragment targeting a target selected from the group consisting of: Her2, DLL3, CLDN6, EGFR, TGFβ, BCMA, B7H6, GUCY2C, CD38, CD123, CD19, CD20, CD22, B7-H3, GPC3, CD73, PMSA, CD28, 4-1BB, OX40, CD40, CD27, CTLA4, PD1, PDL1, BCMA, GLP-1, Trop2, TIGIT, LAG-3, FGL1, TLR7.

In another preferred embodiment, the recombinant protein (or polypeptide) comprises a fusion protein.

In another preferred embodiment, the fusion protein includes a multispecific antibody and a chimeric antibody.

In another preferred embodiment, the functional domain that enhances protein physicochemical properties or druggability includes Fc segment, anti-albumin nanobody (HLE), and albumin binding domain (ABD).

In the fourth aspect of the present invention, it provides an isolated polynucleotide encoding the mutant CD3 antibody of the first aspect of the present invention, or the multispecific antibody of the second aspect of the present invention.

In the fifth aspect of the present invention, it provides a vector comprising the polynucleotide of the fourth aspect of the present invention.

In the sixth aspect of the present invention, it provides a genetically engineered host cell comprising the vector of the fifth aspect of the present invention or having the polynucleotide of the fourth present aspect of the invention integrated into its genome.

In the seventh aspect of the present invention, it provides a method for preparing the multispecific antibody of the second aspect of the present invention, comprising the steps of:
(i) culturing the host cell of the sixth aspect of the invention under a suitable condition, thereby obtaining a mixture comprising the multispecific antibody of the second aspect of the invention; and
(ii) purification and/or separation of the mixture obtained in step (i) , thereby obtaining the multispecific antibody of the second aspect of the present invention.

In the eighth aspect of the invention, it provides a pharmaceutical composition comprising:
(I) the multispecific antibody of the second aspect of the invention; and
(II) a pharmaceutically acceptable carrier.

In another preferred embodiment, the pharmaceutical composition also comprises:
(b) other biologically active drugs, such as those used to treat tumors.

In another preferred embodiment, the pharmaceutical composition includes single drugs, compound drugs, or synergistic drugs.

In another preferred embodiment, the administration method of the pharmaceutical composition is selected from the group consisting of: subcutaneous injection, intradermal injection, intramuscular injection, intravenous injection, intraperitoneal injection, microneedle injection, oral administration, or oral and nasal spray and atomization inhalation.

In another preferred embodiment, the administration method of the pharmaceutical composition is: after co-culturing the pharmaceutical composition and immune cells (such as dendritic cells, natural killer cells, lymphocytes, mononuclear/macrophages, granulocytes, etc.), separating immune cells for *in-vivo* reinfusion.

In another preferred embodiment, the dosage form of the pharmaceutical composition is selected from the group consisting of liquid, solid, or gel.

In another preferred embodiment, the pharmaceutical composition is used for anti-tumor treatment.

In the ninth aspect of the present invention, it provides an immunoconjugate, which comprises:
(a) the multispecific antibody of the second aspect of the invention; and
(b) a coupling moiety selected from the group consisting of: a detectable label, a drug, a toxin, a cytokine, a radionuclide, an enzyme, and a combination thereof.

In another preferred embodiment, the components (a) and (b) are operatively connected.

In another preferred embodiment, the coupling moiety is selected from the group consisting of: a fluorescent or luminescent label, a radioactive label, MRI (magnetic resonance imaging) or CT (electronic computer tomography) contrast agent, or an enzyme capable of producing detectable products, a radionuclide, a biological toxin, a cytokine (such as IL-2), an antibody, an antibody Fc fragment, an antibody scFv fragment, a gold nanoparticle/nanorod, a viral particle, a liposome, a nanomagnetic particle, a prodrug activating enzyme (such as DT-cardiomyolase (DTD) or biphenyl hydrolase-like protein (BPHL)), or a nanoparticle in any form, etc.

In the tenth aspect of the present invention, it provides a use of the mutant CD3 antibody or the modified mutant CD3 antibody of the first aspect of the present invention, the multispecific antibody of the second aspect of the present invention, or the immunoconjugate of the ninth aspect of the invention for preparing (a) a detection reagent or kit; and/or (b) preparing a pharmaceutical composition for preventing and/or treating a cancer, tumor, or autoimmune disease.

In another preferred embodiment, the autoimmune disease includes but is not limited to: systemic lupus erythematosus (SLE), Sjogren's syndrome, scleroderma, rheumatoid arthritis (RA), juvenile idiopathic arthritis, graft versus host disease, dermatomyositis, type I diabetes, Hashimoto's thyroiditis, Graves' disease, Addison's disease, celiac disease, Crohn's disease, pernicious anemia, pemphigus vulgaris, vitiligo, autoimmune hemolytic anemia, idiopathic thrombocytopenic purpura, giant cell arteritis, myasthenia gravis, and multiple sclerosis (MS) (for example, relapsing remitting multiple sclerosis (RRMS)). In the eleventh aspect of the present invention, it provides a method for preventing or treating tumor, wherein the multispecific antibody of the second aspect of the present invention, the pharmaceutical composition of the eighth aspect of the present invention, or the immunoconjugate of the ninth aspect of the present invention is administered to a subject in need.

In another preferred embodiment, the tumors include but are not limited to: breast cancer, liver cancer, stomach cancer, colorectal cancer, leukemia, lung cancer, kidney cancer, small intestine cancer, prostate cancer, colorectal cancer, prostate cancer, cervical cancer, lymphoma, bone cancer, adrenal tumor, or bladder cancer.

It is to be understood that the various technical features of the present invention mentioned above and the various technical features specifically described hereinafter (as in the Examples) may be combined with each other within the scope of the present invention to constitute a new or preferred technical solution, which will not be repeated one by one herein, due to space limitations.

### Description of the drawings

Figure 1 shows the structure and capping diagram of the bispecific antibody of the present invention. Wherein, "-" and "+" represent the charges of the knot into hole (KTH).
Figure 2 shows the results of ELISA detection of the binding affinity between CD3-Her2 TMEAbody (tumor micro-environment activated antibody) and human CD3 (2A) or cynomolgus CD3 (2B). Wherein, CD3-Her2 WT is a wild-type anti-Her2/anti-CD3 bispecific antibody, CD3-Her2 Mutation is a control, CD3-Her2+20k is the anti-Her2/anti-CD3 TMEAbody in the blocked state, CD3-Her2+20k activated is the anti-Her2/anti-CD3 TMEAbody in the activated state.
Figure 3 shows that CD3-Her2 TMEAbody blocks the activation of CD8 T cells in PBMCs (T cell activation induced by bispecific antibody in PBMCs). Wherein, CD3-Her2 WT is a wild-type anti-Her2/anti-CD3 bispecific antibody, CD3-Her2 Mutation is a bispecific antibody comprising anti mutant CD3 antibody, CD3-Her2+20k is the anti-Her2/anti-CD3 TMEAbody in the blocked state, and CD3-Her2+20k activated is the anti-Her2/anti-CD3 TMEAbody in the activated state.
Figure 4 shows the results of RTCC effect evaluation for CD3-Her2 TMEAbody. CD3-Her2+20k is anti-HER2/anti-CD3 TMEAbody in the blocked state, and CD3-Her2+20k activated is anti-HER2/anti-CD3 TMEAbody in the activated state.
Figure 5 shows the *in vivo* characterization of CD3-Her2 TMEAbody in a mouse tumor model. Wherein, CD3-Her2 WT is the bispecific antibody that does not comprise mutant CD3 antibody, and CD3-Her2+20k is the anti-Her2/anti-CD3 TMEAbody in the blocked state.

### EMBODIMENTS

After extensive and intensive research, the inventors of the present invention unexpectedly discovered for the first time that the mutant CD3 antibody obtained by mutating the specific amino acid in the CDRs of the variable regions of wild-type CD3 antibody to Cys and the modified mutant CD3 antibody obtained by chemically coupling it to a blocker could reduce the binding activity of CD3 and achieve specific activation in tumor microenvironment. The bispecific antibody constructed by the modified mutant CD3 antibody and antigen fragments that bind to other target molecular protein (such as anti-HER2 scFv) can be activated and stimulate anti-tumor immune responses in the tumor microenvironment, significantly reducing cytokine storms while greatly increasing the dosage of CD3 antibody. On this basis, the present invention has been completed.

### Terms

Before describing the present invention, it should be understood that the present invention is not limited to the specific methods and experimental conditions described as the methods and conditions can change. It should also be understood that the terms used herein are for the purpose of describing a specific embodiment only, and are not intended to be limiting. The scope of the invention will only be limited by the appended claims.

Unless otherwise defined, all technical and scientific terms used herein have the same meaning as commonly understood by one of ordinary skill in the art to which this invention belongs. As used herein, the term "about", when used in reference to a specifically recited number, means that the value can vary by no more than 1% from the recited value. For example, as used herein, the expression "about 100" includes 99 and 101 and all values between 99 and 101 (for example, 99.1, 99.2, 99.3, 99.4, etc.).

Although any methods and materials similar or equivalent to those described herein can be used in the practice or test of the present invention, the preferred methods and materials are exemplified herein.

### Antibody

Typically, "antibody" is also referred to as "immunoglobulin" which may be a natural or conventional antibody, wherein the two heavy chains are connected to each other through disulfide bonds and each heavy chain is connected to the light chain through disulfide bonds. There are two types of light chains, λ (l) and κ (k). There are five main heavy chain types (or isotypes) that determine the functional activity of antibody molecules: IgM, IgD, IgG, IgA and IgE. Each chain contains different sequence domains. The light chain includes two domains or regions, i.e., a variable domain (VL) and a constant domain (CL). The heavy chain includes four domains, a heavy chain variable region (VH) and three constant regions (CH1, CH2, and CH3, which are collectively referred to as CH). Both the variable regions of light chain (VL) and heavy chain (VH) determine the binding, recognition and specificity of the antigen. The constant domain of the light chain (CL) and the constant region of the heavy chain (CH) impart important biological properties such as antibody chain binding, secretion, transplacental mobility, complement binding, and binding with the Fc receptor (FcR). The Fv fragment is an N-terminal portion of the immunoglobulin Fab fragment and is composed of variable portions of a light chain and a heavy chain. The specificity of the antibody depends on the structural complementary between the antibody binding site and the antigen determination region. The antibody binding site consists of residues from highly variable regions or complementary determining regions (CDRs). Occasionally, residues from non-highly variable or frame regions (FRs) affect the structure of overall domain and in turn affect the binding site. Complementary determining regions or CDRs refer to amino acid sequences that collectively define binding affinity, and specificity of the natural Fv region of natural immunoglobulin binding sites. The light and heavy chains of the immunoglobulin each have three CDRs, which are separately referred to as CDR1-L, CDR2-L, CDR3-L, CDR1-H, CDR2-H, and CDR3-H. The antigen binding sites of a conventional antibody thus include six CDRs comprising a set of CDRs from each v region of heavy chain and light chain.

As used herein, the term "single domain antibody" and "nanobody" have the same meaning, and refer a single domain antibody consisting only of one heavy chain variable region constructed by cloning the variable region of an antibody heavy chain. The single domain antibody is the smallest antigen-binding fragment with full function. Typically, the antibody with natural deletion of light chain and heavy chain constant region 1 (CH1) is first obtained, and the variable region of the antibody heavy chain is cloned, and a single domain antibody consisting only of one heavy chain variable region is constructed.

As used herein, the term "variable" means that antibodies are different from each other in terms of sequence in certain parts of variable regions, which is responsible for the binding and specificity of various specific antibody to their specific antigen. However, the variability is not distributed evenly throughout the variable regions of an antibody. It is concentrated in three segments called complementarity determining regions (CDRs) or hypervariable regions in the light and heavy chain variable regions. The conserved parts of variable regions are called framework regions (FRs). Each of the variable regions of naturally occurring heavy and light chains comprises four FR regions, which are generally in a β-sheet configuration, joined by the three CDRs forming a linking loop, and in some cases, may form a partial β-sheet structure. The CDRs in each chain are closely linked together via the FR regions, and together with the CDRs of the other chain, form the antigen binding site of an antibody (see Kabat et al., NIH Publ. No. 91-3242, Vol. I, pp. 647-669 (1991)). The constant regions are not directly involved in the binding of an antibody to an antigen, however, they exhibit different effector functions, for example, involved in the antibody-dependent cytotoxicity of an antibody.

As used herein, the term "framework region" (FR) refers to the amino acid sequence inserted between the CDRs, i.e., refers to the more conservative portions in the immunoglobulin light chain and heavy chain variable region between different immunoglobulins in a single species. The light chain and heavy chain of the immunoglobulin each has four FRs, called FR1-L, FR2-L, FR3-L, FR4-L and FR1-H, FR2-H, FR3-H, FR4-H, respectively. Accordingly, the light chain variable domain can thus be referred to as (FR1-L)-(CDR1-L)-(FR2-L)-(CDR2-L)-(FR3-L)-(CDR3-L)-(FR4-L) and the heavy chain variable domain can thus be referred to as (FR1-H)-(CDR1-H)-(FR2-H)-(CDR2-H)-(FR3-H)-(CDR3-H)-(FR4-H). Preferably, the FR of the present invention is human antibody FR or a derivative thereof, and the derivative of the human antibody FR is essentially identical to the naturally occurring human antibody FR, i.e., has a sequence identity of 85%, 90%, 95%, 96%, 97%, 98% or 99%.

With the acknowledge of the amino acid sequences of CDRs, those skilled in the art can easily determine the framework regions FR1-L, FR2-L, FR3-L, FR4-L and/or FR1-H, FR2-H, FR3-H, FR4-H.

As used herein, the term "human frame region" is a frame region that substantially same (about 85% or more, specifically is 90%, 95%, 97%, 99% or 100%) as the frame region of the naturally occurring human antibody.

As used herein, the term "monoclonal antibody" or "mAb" refers to an antibody molecule having a single amino acid composition that targets a specific antigen, and is not to be understood to produce the antibody by any particular method. The monoclonal antibody can be produced by a single clone of B cell or hybridoma, but can also be recombinant, i.e., produced by protein engineering.

As used herein, the term "antigen" or "target antigen" is a molecular or portion of a molecule capable of being bound by an antibody or antibody-like binding protein. The term further refers to a molecular or portion of a molecule that can be used in animals to produce an antibody capable of binding to the epitope of the antigen. The target antigen can have one or more epitopes. For each target antigen recognized by antibody or antibody-like binding protein, the antibody-like binding protein can compete with the full antibody that recognize target antigen.

As used herein, the term "affinity" is theoretically defined by the equilibrium association between the full antibody and antigen. The affinity of the bifunctional antibody of the present invention can be evaluated or determined by the KD value (dissociation constant) (or another assay), such as by Bio-Layer Interferometry (BLI), measured with the Fortebiored96 instrument.

As used herein, the term "EC50", also known as the half maximal effect concentration, refers to the antibody concentration that causes 50% maximum effect.

As used herein, the term "Knobs-in-holes (KIH)" refers to the currently mature technique of Fc segment heterodimerization. For example, Knobs structure (T366W) is formed by replacing a smaller threonine (T) at position 366 in the heavy chain CH3 region with a larger tryptophan (W), and holes structure (Y407T) is formed by muting a larger tyrosine (Y) at position 407 in the CH3 region of the other antibody heavy chain to a smaller threonine (T). The KIH structure relies on the decrease of steric hindrance and the formation of covalent disulfide bonds in the hinge region to promote the dimerization of heterologous heavy chain. At present, the commonly used knobs-in-holes structure of Fc segment involves improving the Y407T single point mutation of heavy chain hole to 3-point mutations (T366S, L368A and Y407V) to obtain a stable "1+3" knobs-in-holes structure, or further adding S354C site mutation on the heavy chain Knob and S349C site mutation on the heavy chain hole.

### CD3 and antibody thereof

CD3 (Cluster of differentiation 3) T cell co-receptor is a polyprotein composed of four different polypeptide chains called ε, γ, δ, and ζ chains. The CD3 complex acts as a signaling module for T cell receptor that non-covalently bind to the antigen binding a/b chain of the T cell receptor (TCR). Anti-human CD3 antibody can bind to CD3 molecule on the surface of T cells and initiate T cell signaling cascade, thereby unspecifically causing T cell killing.

As used herein, "the mutant CD3 antibody of the present invention" refers to a CD3 antibody mutant wherein one or more mutations are introduced in the complementarity determining regions (CDR regions) of the heavy chain variable region and/or light chain variable region relative to the wild-type CD3 antibody sequences, wherein the mutations are selected from the group consisting of:
a mutation where one or more amino acids are replaced with cysteine (Cys) at the position of M34, S53, Y61, D68, F104, W111, or F112 of the heavy chain variable region; and/or at the position of Y34, N54, K55, R56, W93, Y94, or N96 of light chain variable region.

### Tumor microenvironment activated antibody

As used herein, "the masked antibody of the present invention", "the tumor microenvironment activated antibody of the present invention", and "the TMEAbody of the present invention" can be used interchanged, referring to a derived antibody comprising the modified mutant CD3 antibody or mutant CD3 antibody (CD3 antibody mutant) of the first aspect of the present invention, wherein the derived antibody is a modified biomolecule (comprising a blocker, a linker peptide cleavable under pathological conditions) that is only activated in the pathological microenvironment, such as in the tumor microenvironment or inflammatory site. Through releasing the biomolecules at the pathological site, the targeting potency of the biomolecules is improved, drug resistance can be overcome and toxicity can be reduced (such as reducing the toxicity of CD3 antibody).

It is understood that the masked antibodies of the present invention include: masked antibodies that have not been activated in the pathological microenvironment and masked antibodies that have been activated in the pathological microenvironment (e.g., activated biomolecules obtained through cleaving the cleavable ligand peptide by proteolytic enzyme in the tumor microenvironment, and releasing the blocker).

Preferably, the modified mutant CD3 antibody of the present invention has reduced binding ability to CD3 in the inactive state (but *in vitro* experiment show that it still retains a certain ability to activate T cell); and restores the T cell activation ability in the activated state to a level comparable to or better than that of the wild-type CD3 antibody.

In cancer patients, tumor cells or antigen-presenting cells (APCs) carrying tumor antigen partially or completely inhibit the host's immune killing of the tumor by binding to T cells. However, the tumor microenvironment activated antibody of the present invention is activated and released by proteolytic enzyme, especially asparagine endopeptidase or granzyme, or under acidic conditions, in the pathological microenvironment.

The bispecific antibody used in the present invention may include an antibody antigen binding region and optionally a cytokine. The antigen binding domain of the bispecific antibody can be selected from: HER2, CD19, EGFR, CD22, CD3, TROP2, glycoprotein NMB, guanylate cyclase C, CEA, CD79b, PSMA, ENPP3, mesothelin, CD138, NaPi2b, CD56, CD74, FOLR1, DLL3, CEACAM5, CD142, SLAMF7, CD25, SLTRK6, CD37, CD70, AGS-22, C4-4A, FGFR2, Ly6E, MUC16, BCMA, pCadherin, Ephrin-A, LAMP1, MUC1, CD19, PDL1, HER2, NY-ESO-1, BCMA, MUC1, CD20, CD23, ROR1, CD123, CD33, CD44v6, CD174, CD30, CD133, cMet, EGFR, FAP, EphA2, GD2, GPC3, IL-13Ra2, LewisY, mesothelin, SS1, CEA, CD171, EGFR, EGFRvIII, VEGFR2, NY-ESO-1, MUC-1, and MAGE-A3, or can be selected from the antigen binding domain of any antibody mentioned in the present invention. In some embodiments, a fusion protein may be a bispecific antibody containing a binding domain targeting an antigen selected from the following group: HER2, CD19, EGFR, CD22, CD3, TROP2, glycoprotein NMB, guanylate cyclase C, CEA, CD79b, PSMA, ENPP3, mesothelin, CD138, NaPi2b, CD56, CD74, FOLR1, DLL3, CEACAM5, CD142, SLAMF7, CD25, SLTRK6, CD37, CD70, AGS-22, C4-4A, FGFR2, Ly6E, MUC16, BCMA, pCadherin, Ephrin-A, LAMP1, MUC1, CD19, PDL1, HER2, NY-ESO-1, BCMA, MUC1, CD20, CD23, ROR1, CD123, CD33, CD44v6, CD174, CD30, CD133, cMet, EGFR, FAP, EphA2, GD2, GPC3, IL-13Ra2, Lewis Y, mesothelin, SS1, CEA, CD171, EGFR, EGFRvIII, VEGFR2, NY-ESO-1, MUC-1, and MAGE-A3, or may be selected from the antigen-binding domain of any of the antibodies mentioned in the present invention. Preferably, a bispecific antibody is a single-stranded bispecific antibody that contains two scFvs from the same or different antibodies. In a specific embodiment, a bispecific antibody comprising a CD3 binding domain is provided.

In some embodiments, a bispecific antibody is an antibody-cytokine fusion protein that comprises an antibody or a functional fragment of an antibody and a cytokine, wherein the cytokine may be selected from: IL-2, IL-7, IL-10, IL-11, IL-12, IL-15, IL-21, IFN-α, IFN-β, IFN-γ, G-CSF, GM-CSF, TNF-α, TRAP, and TRAIL.

The antibody used in the present invention may be a known antibody or functional fragment thereof in this field. For example, the antibody or functional fragment thereof can be selected from: anti-Her2 antibody, anti-EGFR antibody, anti-VEGFR antibody, anti-CD20 antibody, anti-CD33 antibody, anti-PD-L1 antibody, anti-PD-1 antibody, anti-CTLA-4 antibody, anti-TNF alpha antibody, anti-CD28 antibody, anti-4-1BB antibody, anti-OX40 antibody, anti-GITR antibody, anti-CD27 antibody, anti-b-CD40 antibody, or anti-ICOS antibody, anti-CD25 antibody, anti-CD30 antibody, anti-CD3 antibody, anti-CD22 antibody, anti-CCR4 antibody, anti-CD38 antibody, anti-CD52 antibody, anti-complement C5 antibody, anti-RSV F protein, anti-GD2 antibody, anti-GITR antibody, anti-glycoprotein receptor lib/Illa antibody, anti-ICOS antibody, anti-IL2R antibody, anti-LAG3 antibody, anti-Integrinα4 antibody, anti-IgE antibody, anti-PDGFRa antibody, anti-RANKL antibody, anti-SLAMF7 antibody, anti-LTIGIT antibody, anti-TIM-3 antibody, anti-VEGFR2 antibody, anti-VISTA antibody.

Within the scope of the present invention, the meaning and structure of cytokines are as generally defined in this field. It typically involves a class of small proteins with broad biological activity, whose synthesis and secretion are achieved by stimulating immune cells such as monocyte, macrophage, T cell, B cell, NK cell, etc., or non-immune cell such as endothelial cell, epidermal cell, fibroblast, etc. Cytokines can regulate cell proliferation, differentiation, function, and immune response by binding to corresponding receptor. Appropriate cytokines include interleukin, interferon, tumor necrosis factor, colony-stimulating factor, chemokine, growth factor, etc.

Exemplary cytokines include, but are not limited to, IL-2, IL-7, IL-10, IL-12, IL-15, IL-21, IFN-α, IFN-β, IFN-γ, G-CSF, GM-CSF, TNF-α, TRAP, and TRAIL.

Specifically, the present invention provides a multispecific antibody, preferably a bispecific antibody, comprising:
a first combination element D1; and
a second combination element D2;
wherein,
D1 specifically binds to the target molecule CD3 protein; and preferably is the mutant CD3 antibody or a derivative antibody thereof (modified mutant CD3 antibody) of the first aspect of the present invention;
D2 specifically binds to a target molecular protein selected from the group consisting of: Her2, DLL3, and CLDN6.

Wherein, D1 comprises a Fab segment of the CD3 antibody, D1 comprises a heavy chain variable region and a light chain variable region, and the heavy chain variable region comprises the following three complementary determining regions (CDRs):
VH-CDR1 as shown in SEQ ID NO.1,
VH-CDR2 as shown in SEQ ID NO.2, and
VH-CDR3 as shown in SEQ ID NO.3; and
the light chain variable region comprises the following three complementary determining regions (CDRs):
   VL-CDR1 as shown in SEQ ID NO.4,
   VL-CDR2 as shown in SEQ ID NO.5, and
   VL-CDR3 as shown in SEQ ID NO.6.

In another preferred embodiment, D1 and D2 are connected by a linker peptide.

In another preferred embodiment, the bispecific antibody further comprises an Fc segment.

In a specific embodiment, it provides a CD3-Her2 TMEAbody (tumor microenvironment activated antibody) comprising a first binding element D1 and a second binding element D2. Wherein, D1 is preferably a modified mutant CD3 antibody, and D2 is preferably a scFv of an antibody targeting HER2.

In another preferred embodiment, the sequence of the scFv of an antibody targeting HER2 is shown in SEQ ID NO.

12:EVQLVESGGGLVQPGGSLRLSCAASGFNIKDTYIHWVRQAPGKGLEWV
ARIYPTNGYTRYADSVKGRFTISADTSKNTAYLQMNSLRAEDTAVYYCSR
WGGDGFYAMDYWGQGTLVTVSSGGGGSGGGGSGGGGSDIQMTQSPSSL
SASVGDRVTITCRASQDVNTAVAWYQQKPGKAPKLLIYSASFLYSGVPSR
FSGSRSGTDFTLTISSLQPEDFATYYCQQHYTTPPTFGQGTKVEIK

Further, the modified mutant CD3 antibody has the following structure:

Ab-(P-R1)n

wherein,
Ab is a CD3 antibody mutant;
R1 is each independently a blocker;
P is a cleavable linker peptide;
n is a positive integer from 1 to 5;
"-" is a chemical bond or connector or linker.

Preferably, the blocker R4 is site-specifically connected to an amino acid site selected from the group consisting of the CD3 antibody mutant through a cleavable linker peptide P: a position corresponding to M34, S53, Y61, D68, F104, W111 or F112 of the complementary determining regions of the heavy chain variable region of wild-type CD3 antibody; and/or a position corresponding to Y34, N54, K55, R56, W93, Y94, or N96 of the complementary determining regions of the light chain variable region of wild-type CD3 antibody; and preferably is connected to the position of Y61 of the complementary determining regions of the heavy chain variable region.

The preferred mutation site includes VH-Y61C in VH and VL-Y34C in VL. Wherein, the VH-CDR2 of the CD3 antibody mutant corresponding to the mutation site of VH-Y61C is: RIRSKYNNYATCYADSVKD (SEQ ID NO. 9).

Within the scope of the present invention, one or more (e.g., up to 5 or 3) amino acid residues in the amino acid sequence of D1 biomolecule are mutated into cysteine, and covalently linked to the blocker (P-R1) through thiol bonds of cysteine. For example, one or two amino acid residues in a biomolecule of interest is mutated into cysteine to attach to a functional group. For an antibody, its mutation site can be in the complementary determining region or non-complementary determining region of the variable region.

Preferably, a mutation is a substitution mutation. More preferably, the mutation occurs in the variable region of the antibody light chain. Typically, a mutant can be prepared first and then tested for its binding activity to the corresponding antigen. If the mutant maintains binding activity of 70% or more, preferably 80% or more, and preferably 90% or more compared to the wild-type antibody, the amino acid residue at the mutant site is considered acceptable to be mutated to cysteine and covalently bonded to the functional group. Alternatively, in some embodiments, an amino acid residue at the mutant site is considered acceptable to be mutated to cysteine and covalently bonded to a functional group if the conjugate produced by linking the mutant to R4 retains 80% or more, preferably 90% or more, and preferably 95% or more binding activity.

As used herein, the term "linking peptide" refers to one or more amino acid residues inserted into the immunoglobulin domain that provide sufficient mobility for the light and heavy chain domains to fold into an exchange double variable region immunoglobulin.

Examples of suitable connector include single glycine (Gly) or serine (Ser) residues, and the identification and sequence of amino acid residues in the connector may vary depending on the type of secondary structural element to be implemented in the connector. A preferred connector is as follows: GS, (G₄S) n, where n is an integer from 1 to 4.

The dual antibody of the present invention includes not only an intact antibody, but also the fragments of the antibody having an immunological activity or a fusion protein formed by the antibody and another sequence. Therefore, the present invention also includes fragments, derivatives and analogs of the antibody. As used herein, the terms "fragments", "derivatives" and "analogs" refer to the polypeptides basically maintaining the same biological function or activity of the antibody of the present invention. The polypeptide fragments, derivatives or analogs of the present invention may be (i) a polypeptide with one or more conservative or non-conservative amino acid residues (preferably the conservative amino acid residues) being substituted, while such substituted amino acid residues may or may not be encoded by genetic code, or (ii) a polypeptide having substituted group(s) in one or more amino acid residues, or (iii) a polypeptide formed by fusion of the matured polypeptide with another compound (such as the compound that prolongs the half-life of the polypeptide, such as polyethylene glycol), or (iv) a polypeptide formed with additional amino acid sequence fused to said polypeptide sequence (such as, leader sequence, secretion sequence, or a sequence or a protein sequence used to purify the polypeptide, or a fusion protein formed with 6His tag). According to the subject application, these fragments, derivatives and analogs are within the scope commonly known by the skilled person.

The dual antibody of the present invention refers to an antibody having anti-CD3 and anti-Her2 activity. The term also includes variant forms of the antibody comprising two of the above-mentioned structures, which have the same function as the dual antibody of the present invention. These variant forms include, but are not limited to, deletions of one or more amino acids (typically 1-50, preferably 1-30, more preferably 1-20, most preferably 1-10), insertions and/or substitutions, and the addition of one or several amino acids (typically at most 20, preferably at most 10, more preferably at most 5) at the C-terminus and/or N-terminus. For example, in the art, the protein's functions are usually unchanged when an amino acid is substituted by a similar or analogous one. Also, for example, the addition of one or several amino acids at the C-terminus and/or the N-terminus will not normally alter the function of the protein. The term also includes active fragments and active derivatives of the dual antibody of the present invention.

The variant forms of the dual antibody include homologous sequences, conserved variants, allelic variants, natural mutants, induced mutants, proteins encoded by a DNA capable of hybridizing to the coding DNA of the antibody of the present invention under high or low stringency conditions, and a polypeptide or protein obtained using an antiserum against the antibody of the present invention.

In the present invention, "the conservative variant of the dual antibody of the present invention" refers to a polypeptide formed by substitution of at most 10, preferably at most 8, more preferably at most 5, and most preferably at most 3 amino acids with amino acids having similar or analogous property, as compared to the amino acid sequence of the dual antibody of the present invention. These conservative variant polypeptides are preferably formed by carrying out the amino acid substitution according to Table A.

**Table A**

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

### Coding nucleic acid and expression vector

The present invention also provides a polynucleotide molecule encoding the antibody or a fragment thereof or a fusion protein thereof. The polynucleotides of the present invention can be in a form of DNA or RNA. DNA forms include cDNA, genomic DNA, or synthetic DNA. DNA can be single-stranded or double-stranded. DNA can be the coding strand or the non-coding strand. The polynucleotides encoding the mature polypeptides of the present invention comprise coding sequences encoding only the mature polypeptide; coding sequences of the mature polypeptide and various additional coding sequences; coding sequences of the mature polypeptide (and optionally additional coding sequences), and non-coding sequences.

The term "polynucleotide encoding a polypeptide" may include a polynucleotide that encodes the polypeptide, or a polynucleotide that also includes additional coding and/or non-coding sequences.

The nucleic acid (and the combination of nucleic acid) of the present invention can be used to generate the recombinant antibody of the present invention in an appropriate expression system.

The present invention also relates to polynucleotides that hybridize to the sequences as described above and having at least 50%, preferably at least 70%, more preferably at least 80% identical between the two sequences. In particular, the present invention relates to polynucleotides that can hybridize to the polynucleotides of the present invention under stringent conditions. In the present invention, "stringent conditions" means: (1) hybridization and elution at lower ionic strength and higher temperature, such as 0.2 x SSC, 0.1% SDS, 60°C; or (2) hybridization adding a denaturant, such as 50% (v/v) formamide, 0.1% calf serum/0.1% Ficoll, 42°C, or the like; or (3) hybridization only occurs when the identity between the two sequences is at least 90%, more preferably 95% or more. And the polypeptide encoded by the hybridizable polynucleotide has the same biological function and activity as the mature polypeptide.

The whole length of the nucleotide sequence or the fragment thereof of the antibody of the present invention can be obtained via PCR amplification, recombinant method or artificial synthesis. One feasible method is to synthesize relevant sequences by artificial method, especially when the fragment is short in length. Usually, several small fragments are synthesized first, and then are linked together to obtain a fragment with a long sequence. In addition, the sequence coding the heavy chain and the expression label (e.g., 6His) can be fused together to form a fusion protein.

Once a relevant sequence is obtained, the relevant sequence can be obtained in bulk using a recombination method. This is usually carried out by cloning the sequence into a vector, transforming a cell with the vector, and then separating the relevant sequence from the proliferated host cell by conventional methods. The biomolecules (nucleic acids, proteins, etc.) involved in the present invention include biomolecules that exist in an isolated form.

At present, a DNA sequence encoding the protein of the present invention (or fragments thereof, or derivatives thereof) can completely be obtained by chemical synthesis. The DNA sequence can then be introduced into a variety of existing DNA molecules (or, for example, vectors) and cells known in the art. In addition, mutations can also be introduced into the protein sequences of the present invention by chemical synthesis.

The present invention further relates to a vector comprising said suitable DNA sequence and a suitable promoter or a control sequence. These vectors can be used to transform suitable host cells to enable them to express protein.

The host cell can be a prokaryotic cell, such as a bacterial cell; or a lower eukaryotic cell, such as a yeast cell; or a higher eukaryotic cell, such as a mammalian cell. Representative examples are: the bacterial cell of *Escherichia coli, streptomactinus, salmonella typhimurium;* fungal cells such as yeast; insect cells of Drosophila S2 or SF9; animal cells of CHO, COS7, 293 cells, etc.

Transformation of host cells with recombinant DNA can be carried out using conventional techniques well known to the skilled in the art. When the host is a prokaryote such as *E. coli,* competent cells capable of absorbing DNA can be harvested after the exponential growth phase and treated with CaCl₂, the steps used are well known in the art. Another method is to use MgCl₂. If necessary, the transformation can also be carried out by means of electroporation. When the host is a eukaryote, the following DNA transfection methods can be used: calcium phosphate coprecipitation method, conventional mechanical method, such as micro-injection, electroporation, liposome packaging, etc.

The obtained transformants can be cultured by a conventional method to express a polypeptide encoded by a gene of the present invention. According to the host cell used, the medium used in the culture may be selected from a variety of conventional media, and the host cell is cultured under conditions suitable for the growth of the host cell. After the host cell has grown to an appropriate cell density, the selected promoter is induced by a suitable method (such as temperature conversion or chemical induction) and the cells are cultured for a further period of time.

In the early culture conditions, the expression level of bispecific antibody can reach 3.9g/L, and the purity is above 97%, and it can metabolize lactic acid well in the culture process.

The recombinant polypeptide in the method above may be included in the cells, or expressed on the cell membrane, or secreted out of the cell. If necessary, the recombinant protein can be separated and purified by various separation methods according to its physical, chemical, and other properties. These methods are well known to those skilled in the art and include, but are not limited to, conventional renaturation treatment, treatment by protein precipitant (such as salt precipitation), centrifugation, cell lysis by osmosis, sonication, supercentrifugation, molecular sieve chromatography (gel chromatography), adsorption chromatography, ion exchange chromatography, high performance liquid chromatography (HPLC), and any other liquid chromatography, and the combination thereof.

The dual antibody of the present invention can be used alone, or can be combined or coupled with a detectable marker (for diagnostic purposes), a therapeutic agent, or any combination of these substances.

Detectable markers for diagnostic purposes include, but are not limited to: fluorescent or luminescent markers, radioactive markers, MRI (magnetic resonance imaging) or CT (electronic computer X-ray tomography technique) contrast agents, or enzymes capable of producing a detectable product.

Therapeutic agents that can be combined or coupled with the antibody of the invention include but are not limited to: 1. Radionuclides; 2. Biotoxin; 3. Cytokines such as IL-2; 4. Gold nanoparticles/nanorods; 5. Virus particles; 6. Liposomes; 7. Magnetic nanoparticles; 8. Tumor therapeutic agents (e.g., cisplatin) or anti-tumor drugs in any form, and so on.

### Pharmaceutical composition

The present invention further provides a composition. Preferably, the composition is a pharmaceutical composition comprising the bispecific antibody of the present invention or an active fragment thereof or a fusion protein thereof, and a pharmaceutically acceptable carrier. In general, these substances may be formulated in a non-toxic, inert and pharmaceutically acceptable aqueous carrier medium, wherein the pH is generally about 5-8, preferably, pH is about 6-8, though the pH value may be varied depending on the nature of the substances to be formulated and the condition to be treated. The formulated pharmaceutical composition may be administered by conventional routes, including (but not limited to): intravenous injection, intravenous drip, subcutaneous injection, topical injection, muscle injection, intratumor injection, intra-abdominal (such as intraperitoneal) injection, intracranial injection, or intra-cavity injection.

The pharmaceutical composition according to the present invention comprises a safe and effective amount (e.g., 0.001-99 wt%, preferably 0.01-90 wt%, preferably 0.1-80 wt%) of the nanobody according to the present invention (or a conjugate thereof) and a pharmaceutically acceptable carrier or excipient. Such carriers include, but are not limited to, saline, buffer solution, glucose, water, glycerin, ethanol or the combination thereof. The pharmaceutical preparation should be matched to the method of administration. The pharmaceutical composition of the present invention can be prepared in the form of injection, for example, prepared by a conventional method using physiological saline or an aqueous solution containing glucose and other adjuvants. Pharmaceutical compositions such as injections and solutions are preferably prepared under sterile conditions. The dosage of active ingredient is therapeutically effective amount, for example from about 10 microgram per kilogram body weight to about 50 milligrams per kilogram body weight per day. Further, the polypeptide of the present invention can also be used in combination with the other therapeutic agents.

In the present invention, the bispecific antibody can be used alone, and the dosage regimen can be adjusted to obtain the best objective response. For example, the drug may be administrated in a single dose, or multiple times over a period of time, or the dose may be reduced or increased proportionately according to the urgency of the treatment situation.

When a pharmaceutical composition is used, a safe and effective amount of the immunoconjugate is administered to a mammal, wherein the safe and effective amount is usually at least about 10 micrograms per kilogram of body weight, and in most cases does not exceed about 50 mg/kg body weight, preferably the dose is about 10 micrograms/kg body weight to about 10 mg/kg body weight. Of course, the particular dose should also depend on various factors, such as the route of administration, patient healthy status, which are well within the skills of an experienced physician.

### The main advantages of the present invention include:

(1) The present invention provides diverse tumor microenvironment activated tumor-microenvironment multispecific antibodies, which is connected through chemical coupling and has diverse activation modes and linker diversity.
(2) The modified mutant CD3 antibody of the present invention increases the dosage of CD3 antibody, reduces toxic side effects (such as cytokine storm), and improves therapeutic efficacy.
(3) The mutant of CD3 antibody of the present invention does not affect the activation of T cell.

The present invention will be further illustrated below with reference to the specific examples. It should be understood that these examples are only to illustrate the present invention, not to limit the scope of the present invention. The conditions of the experimental methods not specifically indicated in the following examples are usually in accordance with conventional conditions as described in e.g., Sambrook et al., Molecular Cloning: A Laboratory Manual (New York: Cold Spring Harbor Laboratory Press, 1989), or according to the conditions recommended by the manufacturers. Percentages and parts are calculated by weight unless otherwise stated.

### Example 1: Analysis of binding activity after CDR mutation in antibody variable region

The humanized amino acid sequences of the anti-CD3 antibody disclosed in US9587021B2 is as shown in SEQ ID NO. 4 and SEQ ID NO. 8, whose DNA sequence was optimized for expression and synthesis in the host (GENEWIZ, Inc., Suzhou, China). The synthetic DNA was digested and attached to a modified pTT5 vector (Biovector) to produce PTT5-anti-CD3. Using PTT5-anti-CD3 as the template, the cysteine mutation was introduced. Primers were designed to replace the codon at the mutation site with the cysteine (Cys) codon. PCR was performed, and the mutant fragment was digested and constructed into a modified pTT5 vector.

The expression host is HEK293T cells (Life Technologies). Before transfection, HEK293T cells were cultured in complete medium containing 10% FBS (Gibico) at 37°C and 5% CO₂. On the day before transfection, cells were seeded at an appropriate density into a 15cm culture dish and the medium was replaced with low IgG FBS (Gibico). After 6 hours of transfection or on the next day, the culture medium was replaced with Freestyle293 (Gibico).

On the day of transfection, when the cells reached a certain confluence, the plasmid expressing the target protein was co-transfected into 293T cells using lipofectamine 2000 (Life Technologies) and PEI (sigma). The antibody expression plasmid includes the heavy and light chains of the antibody. The culture supernatant was collected on the 4^{th} and 6^{th} day after transfection, respectively. The expression and activity of proteins or antibodies were detected, and the protein or antibody was purified.

The highly variable regions in the heavy and light chains of antibody (Ab) constitute the antigen (Ag) binding site of antibody. Because the structure of the antigen binding site is complementary to the antigen epitope, the highly variable regions are also known as the antibody's complementarity determining regions (CDRs).

In the present invention, the activity of each mutant with a point mutation compared to that of its initial antibody is detected by ELISA. Specifically, the 96-well plates were coated with antibody antigen overnight, then blocked with 1%BSA blocking agent (Thermo Fisher) at 37°C for 2 hours, and washed with PBST for 3 times. The corresponding antibody or the corresponding mutant was added for binding at 37°C for 1 hour, and then washed with PBST for 3 times. HRP enzyme-linked anti-human IgG was added for binding at 37°C for 1 hour, and washed with PBST for 3 times. TMB substrate (Solarbio., Inc.) was used to detect absorbance at 450nm. The effect on mutant binding strength was calculated through EC50 mutation/EC50 wild-type.

According to the above method, the binding activity of anti-CD3 antibody SP34 mutant with mutations at different position shown in Table 1 was detected.
Amino acid sequence of heavy chain variable region (VH) of the anti-CD3 antibody disclosed in US9587021B2 (wild-type CD3 antibody) 
EVQLVESGGGLVQPKGSLKLSCAASGFTFNTYAMNWVRQAPGKGLE
WVARIRSKYNNYATYYADSVKDRFTISRDDSQSILYLQMNNLKTEDTAM
YYCVRHGNFGNSYVSWFAYWGQGTLVTVSS (SEQ ID NO. 10)
Amino acid sequence of light chain variable region (VL) 
QAVVTQESALTTSPGETVTLTCRSSTGAVTTSNYANWVQEKPDHLFT
GLIGGTNKRAPGVPARFSGSLIGDKAALTITGAQTEDEAIYFCALWYSNLW
VFGGGTKLTVL (SEQ ID NO. 11)
Amino acid sequence of heavy chain variable region (VH) of anti-CD3 antibody 
EVQLVESGGGLVQPGGSLRLSCAASGFTFSTYAMNWVRQAPGKGLEWV
GRIRSKYNNYATYYADSVKDRFTISRDDSKNSLYLQMNSLKTEDTAVYYCAR
HGNFGNSYVSWFAYWGQGTLVTVSS (SEQ ID NO. 4)
Amino acid sequence of the light chain variable region (VL) of anti-CD3 antibody

QTVVTQEPSLTVSPGGTVTLTCRSSTGAVTTSNYANWVQQKPGQAPRGL
IGGTNKRAPGVPARFSGSLLGDKAALTLLG.AQPEDEAEYFCALWYSNLWVF
GGGTKLTVL (SEQ ID NO. 8)

**Table 1: Effect of mutation location in the heavy/light chain variable region of antibody on binding activity**

| CDR | Location | Primitive amino acids of anti-CD3 antibody | Binding strength of mutants (EC50 mutant/EC50 wild-type) |
|---|---|---|---|
| HCDR1 | VH-34 | M34 | 1.1 |
| HCDR2 | VH-53 | S53 | 1.2 |
| HCDR2 | VH-61 | Y61 | 5.1 |
| HCDR2 | VH-68 | D68 | 2 |
| HCDR3 | VH-104 | F104 | 1.0 |
| HCDR3 | VH-111 | W111 | 2 |
| HCDR3 | VH-112 | F112 | 1.2 |
| LCDR1 | VL-34 | Y34 | 1.2 |
| LCDR2 | VL-54 | N54 | 1 |
| LCDR2 | VL-55 | K55 | 0.9 |
| LCDR2 | VL-56 | R56 | 1.5 |
| LCDR3 | VL-93 | W93 | 1.8 |
| LCDR3 | VL-94 | Y94 | 1 |
| LCDR3 | VL-96 | N96 | 1 |

### Example 2: Analysis of binding activity of anti-CD3 antibody mutant after conjugated with molecule I

As shown in Table 1, antibodies with mutation in the CDRs of light or heavy chains were conjugated with Molecule I from the small molecule library used for conjugation, and their binding activity was compared with that of the wild-type antibody (binding effect of mutant = binding activity of conjugated mutant/ binding activity of wild-type antibody × 100%) to obtain a conjugation method that can provide intermolecular forces and reduce binding activity.

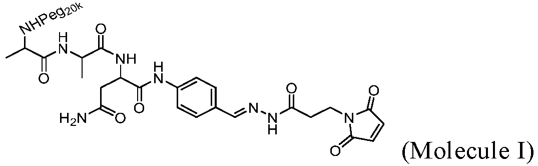

**Table 2: Binding effect of anti-CD3 antibody mutants conjugated with molecule I**

| CDR | Location | Primitive amino acids of anti-CD3 antibody | multiple Blocking fold after conjugation (+20K) |
|---|---|---|---|
| HCDR1 | VH-34 | M34 | 5 |
| HCDR2 | VH-53 | S53 | 3 |
| HCDR2 | VH-61 | Y61 | 400 |
| HCDR2 | VH-68 | D68 | 4 |
| HCDR3 | VH-104 | F104 | 13 |
| HCDR3 | VH-111 | W111 | 6 |
| HCDR3 | VH-112 | F112 | 4 |
| LCDR1 | VL-34 | Y34 | 8 |
| LCDR2 | VL-54 | N54 | 12 |
| LCDR2 | VL-55 | K55 | 5 |
| LCDR2 | VL-56 | R56 | 6 |
| LCDR3 | VL-93 | W93 | 5 |
| LCDR3 | VL-94 | Y94 | 30 |
| LCDR3 | VL-96 | N96 | 2 |

According to Table 2, after mutating M34, S53, Y61, D68, F104, W111, F112 in HCDRs of VH, or Y34, N54, K55, R56, W93, Y94, and N96 in LCDRs of VL of anti-CD3 antibody to C and connecting the antibody to R4, the conjugated mutant will reduce binding efficiency. Wherein, the preferred mutation site includes VH-Y61C in VH and VL-Y34C in VL.

### Example 3: Production and characterization of anti-HER2/anti-CD3 bispecific TMEA antibody

The heavy and light chain sequences of trastuzumab were downloaded from Drug Bank (https://www.drugbank.ca/drugs/DB00072). The CD3 antibody used the sequences of CD3 antibody with single-point mutation (VH-Y61C) screened above.

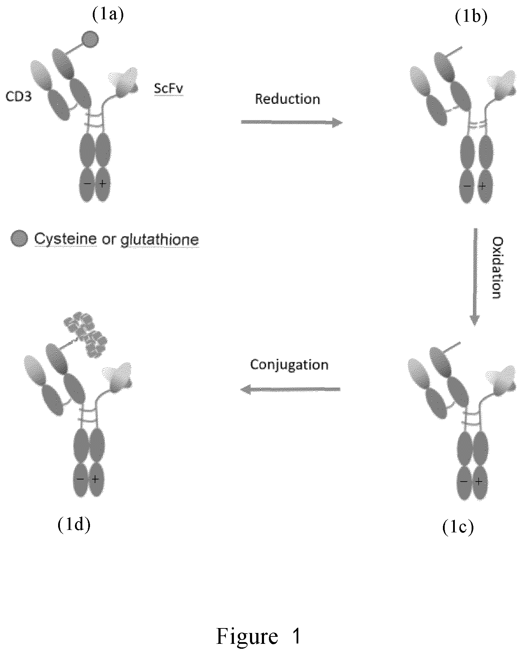

CD3-HER2 antibody was prepared by expression in 293 cells and purification using standard techniques. The cysteine incorporated in antibody preparations initially existed as a disulfide mixed with cysteine or glutathione, and was therefore not initially reactive to the thiol reactive payload. Figure 1 depicts the process of conjugation with a blocker, starting from the treatment (deblocking) of the free thiol of the incorporated Cys to make it fully available for conjugation. Wherein, 1a is the anti-Her2/anti-CD3 bispecific antibody (CD3-HER2 Mutation, with the mutation site VH-Y61C) comprising the anti-CD3 antibody mutant, and 1d is the CD3-Her2 TMEAbody conjugated with Blocker (PEG20K).

The structural formula of the cleavable linker peptide (P) attached to a blocker (R1) is as follows:

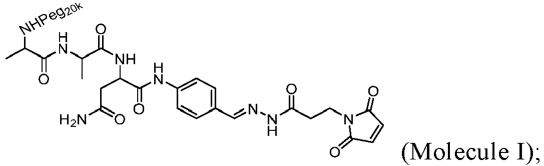

Wherein, the structural formula of R1 (PEG20K) is:

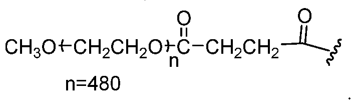

In addition, the effect of different R1 on the binding activity after conjugation was screened:

**Table 3 Effect of different R1 on binding activity after conjugation**

| Heavy/light chain variable region | Mutation site | R1 | Blocking fold after conjugation | Recovery of binding force after activation (%) |
|---|---|---|---|---|
| VH-61 | Y61C | PEG5K | 10 | 100 |
| VH-61 | Y61C | PEG10K | 20 | 100 |
| VH-61 | Y61C | PEG20K | 400 | 100 |
| VH-61 | Y61C | PEG40K | 500 | 100 |

### Example 4: CD3-Her2 TMEAbody reduces binding affinity with CD3

On day 0, a plate was coated with 100 µl/well of 1 µg/ml recombinant human or cynomolgus CD3 Epsilon protein (KACTUS) in PBS at 4°C overnight. On day 1, plate blocking: blocking was performed using 300µl blocking buffer at room temperature for 2 hours, and after blocking, the plate was washed 3 times. Antibody preparation: the test antibody and positive antibody were diluted with dilution buffer to obtain the final concentration: starting from 15 nM, 5 times dilution (7 points + 0 point). 100µl antibody solution was added to the 96-well plate. The plate was thoroughly vibrated and incubated at room temperature for 1 hour. The plated was washed three times. Secondary antibody preparation: the secondary antibody was diluted at 1:5000 in dilution buffer (according to the layout). 100 µl of secondary antibody solution was added to a 96 well plate and left at room temperature for 0.5 hours. The plate was washed for three times. TMB was prepared and 100 µl was added to each well. The plate was incubated at 37°C for 5 minutes until blue appears (incubation time should be the same for each test). The reaction was terminated by 50µl ELISA termination buffer. The plate was gently shaken and the OD value of the plate was measured at 450nm.

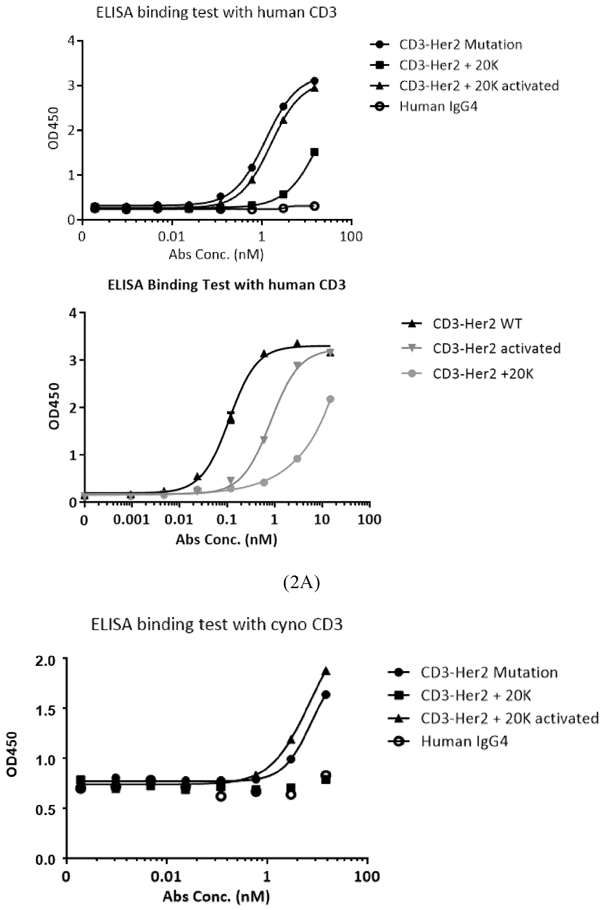

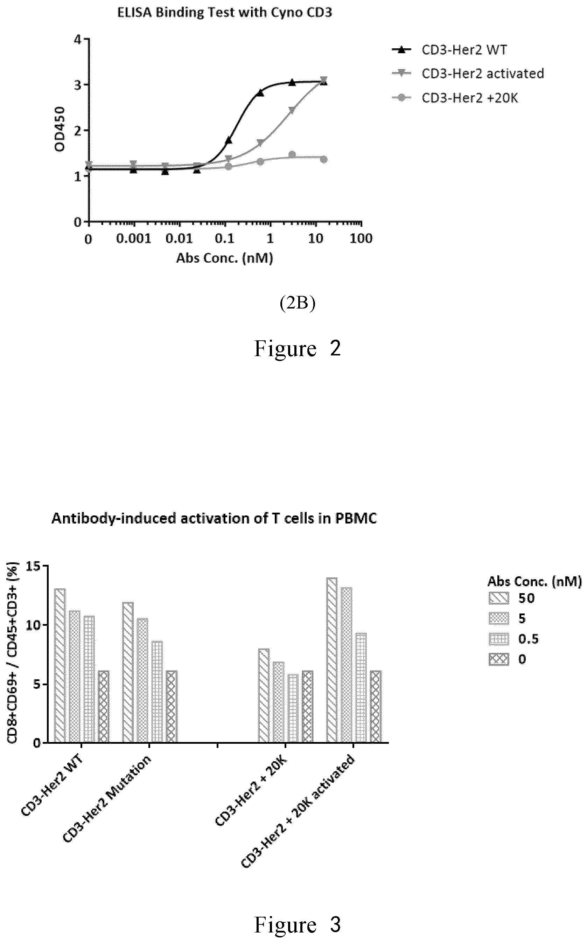

As shown in Figure 2, the binding affinity of CD3-Her2 TMEAbody (CD3-Her2+20k) in the blocked state for recombinant human CD3 (Figure 2A) is lower than that of CD3-Her2 Mutation for recombinant human CD3, while the CD3-Her2 TMEAbody (CD3-Her2+20k activated) in the activated state restored its binding affinity which was comparable to the binding affinity of CD3-Her2 Mutation.

CD3-Her2 Mutation and CD3-Her2+20k activated weakly binds to cynomolgus monkey CD3 (Figure 2B), while CD3-Her2+20k hardly binds to cynomolgus monkey CD3.

### Example 5: CD3-Her2 TMEAbody blocks activation of CD8 T cells

N87 cells were removed from the incubator and digested into single-cell suspension with pancreatic enzyme. 1mL of the single-cell suspension was counted on the VI-Cell cell counter, and then added to the 96-well plate according to the experimental design, with 4×10⁴ cells/100µL per well, and cultured in the incubator at 37°C. After 24 hours, the prepared 5×antibody molecule was added at 50µL/well. hPBMC was counted on CellometerVI-Cell counter, then added into 96-well plate at 8×10⁵ cells/100µL per well. The cell culture plate was cultured in a cell incubator for 4h. The cell suspension from the cell culture plate was mixed and taken into another 96-well plate, and centrifuged at 400×g for 5min. The cell was resuspended in a 100µL buffer containing CD45 monoclonal antibody (HI30), PE-Cyanine7 (eBioscience^{™}, Cat# 25-0459-42), FITC-labeled anti-human CD3 antibody (Clone OKT3) (Biolegend, Cat#317306), APC-labeled anti-human CD8 antibody (Clone: SK1) (Biolegend, Cat#344722), PE-labeled anti-human CD69 antibody (Biolegend, Cat#FN50). After incubation at 4°C for 30min, cells were washed once with buffer, and then suspended in 100µL buffer for detection by flow cytometry (BECKMAN COULTER, CytoFLEX).

According to Figure 3, the mutated bispecific antibody CD3-Her2 Mutation does not affect the activation of T cell. Although CD3-Her2+20k reduces the binding force for CD3, it is still able to maintain a certain activation capacity of T cell, and restores the activation capacity of T cell equivalent to or better than CD3-Her2 WT in the activated state.

### Example 6: Valuation of re-directed T cell cytotoxicity (RTCC) of CD3-Her2 TMEAbody

T cell-mediated tumor cell killing and T cell activation: Target cells were harvested with trypsin EDTA, counted, and examined for viability. The cells were resuspended in respective culture media, with a final concentration of 4×10⁴ cells per milliliter. Then 100 µl of the target cell suspension was transferred into each well of a 96 µl flat bottomed plate. The plate was incubated overnight at 37°C in the incubator to allow cells to adhere to the plate. The next day, PBMC was isolated from the whole blood of a healthy donor. Blood was diluted at 2:1 with PBS and covered on 15 ml of Histopaque-1077 (# 10771, Sigma-Aldrich) in a Leucosep tube and centrifuged continuously at 450 g for 30 min. After centrifugation, band containing cells was collected using a 10 ml pipette and transferred into a 50 ml tube. The tube was filled with PBS up to 50 ml and centrifuged (400 g, 10 min, room temperature). The supernatant was removed and the pellet was resuspended in PBS. After centrifugation (300g, 10 min, room temperature), the supernatant was discarded, the 2 test tubes were combined and the washing step was repeated (centrifugation 350×g, 10 min, room temperature) (using PBMC purchased from other companies). The cells were then re-suspended and the precipitates were pooled in 50 ml PBS for cell counting. After counting, the cells were centrifuged (350g, 10 minutes, room temperature) and resuspended in RPMI containing 2% FBS at a concentration of 6 million cells per milliliter. The culture medium was removed from the target cells and the test antibody diluted in RPMI containing 2% FBS was added. The effector cell solution containing 8 × 10⁵ cells was transferred to each well, resulting in an E: T ratio of 20:1. Target cells were lysed with a lysate buffer to determine maximum release. 48 hours later, LDH release was determined by BioTek, using Cytotoxity Detection KitPLUS (LDH) (Roche, Cat # 4744934001) and ELx800 Absorption Microplate Reader.

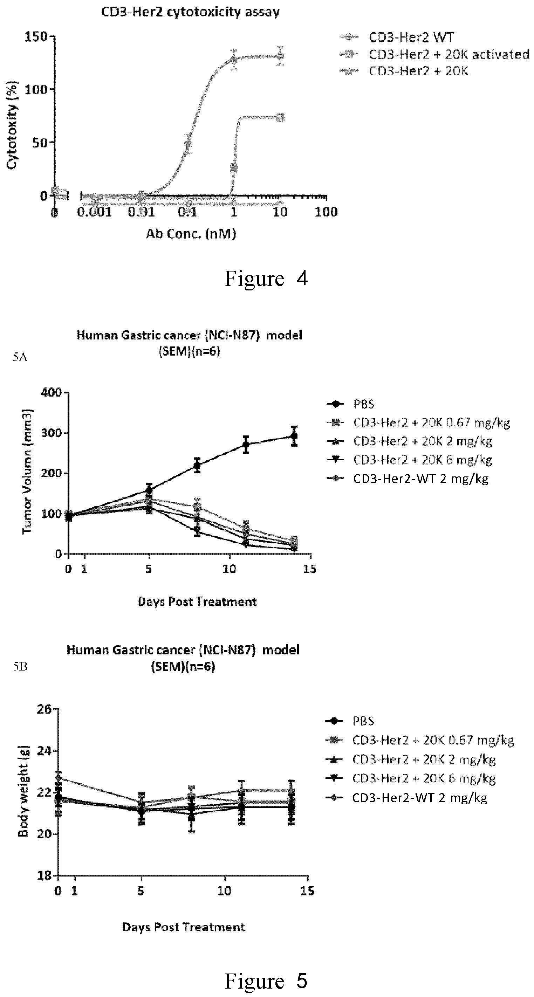

As shown in Figure 4, the CD3-Her2 TMEAbody (CD3-Her2+20k) in the blocked state blocked most of the RTCC efficacy, while the CD3-Her2 TMEAbody (CD3-Her2+20k activated) in the activated state restored some of the RTCC efficacy.

### Example 7: In vivo characterization of CD3-Her2 TMEAbody in a mouse tumor model

Human gastric cancer cells NCI-N87 cells were subcutaneously transplanted into NCG mice to construct an *in vivo* model. NCI-N87 cells were cultured according to the culture conditions, and the cells were collected and counted during the exponential growth period. Each mouse was inoculated with 5×10⁶ human gastric cancer cells NCI-N87 (suspended in 0.1ml basal medium) on the right side, and PBMC cells were injected via tail vein to rebuild the NCG immune system. After vaccination, when the tumor grew to 70-150 mm³ (8 days after tumor loading), mice were randomly divided into groups based on their body weight and tumor volume. The experiment is divided into:
PBS group,
CD3-Her2+20K (0.67 mg/kg) group,
CD3-Her2+20K (2 mg/kg) group,
CD3-Her2+20K (6 mg/kg) group,
CD3-Her2 WT (2 mg/kg) group.

Wherein, CD3-Her2+20K is CD3-Her2 TMEAbody, and CD3-Her2 WT is an anti-Her2/anti-CD3 bispecific antibody that does not contain anti-CD3 antibody mutant.

Treatment began the next day. The mice were weighed twice a week and the tumor volume was measured with the formula: tumor volume =1/2× length × width × width (mm³). Tumor growth inhibition rate (TGI, %) =100%-(Tt-T0)/(Ct-C0) × 100%; Tumor inhibition rate (T/C, %) =(Tt/T0)/(Ct/C0) × 100%. T0 is the tumor volume of the test antibody group during different administration periods; Tt is the tumor volume of the test antibody group during each measurement; C0 is the tumor volume of PBS group during cage administration. CT is the average tumor volume in the PBS group at each measurement.

As shown in Figure 5, CD3-Her2+20K showed significant tumor growth inhibition in NCI-N87 transplanted tumor model in NCG mice immunized by PBMC cells. After 14 days of treatment, the inhibition rates (TGI) of CD3-Her2+20K (0.67 mg/kg) group, CD3-Her2+20K (2 mg/kg) group, CD3-Her2+20K (6 mg/kg) group, and CD3-Her2 WT (2 mg/kg) group were 131.72%, 136.88%, 142.11%, and 134.92%, respectively, and T/C were 11.04%, 7.34%, 3.84%, and 8.56%, respectively. There was no significant change in weight during the treatment period.

As shown in Figure 5, the results indicate that CD3-Her2 TMEAbody (CD3-Her2+20K) can be activated and stimulate anti-tumor immune responses in the tumor microenvironment, and it has similar *in vivo* efficacy as CD3-Her2 WT while increasing the dosage of administration. The specific data of tumor volume on different days are shown in the table below.

**Table 4. Specific data of tumor volume on different days**

| Tumor volume (mm³) | 0 day | 5 days | 8 days | 11 days | 14 days |
|---|---|---|---|---|---|
| PBS | 94.58 | 158.03 | 219.71 | 271.24 | 292.54 |
| CD3-Her2 TMEAbody 0.67mg/kg | 95.37 | 127.17 | 106.18 | 61.02 | 32.57 |
| CD3-Her2 TMEAbody 2 mg/kg | 94.45 | 113.20 | 87.34 | 37.48 | 21.44 |
| CD3-Her2 TMEAbody 6 | 94.60 | 117.89 | 54.92 | 22.09 | 11.23 |
| mg/kg | | | | | |
| CD3-Her2 WT 2 mg/kg | 94.01 | 131.76 | 91.11 | 50.18 | 24.88 |

### Example 8 Toxicokinetic experiments of CD3-Her2 TMEAbody in rhesus monkeys

Three groups were set in this experiment, low-dose test sample group, high-dose test sample group, and positive control group, with one male animal in each group. CD3-Her2-40K, CD3-Her2-20K, or CD3-Her2-M were intravenously infused, and CD3-Her2-WT was administered as the positive control group. Wherein, CD3-HER2-40K is CD3-Her2 TMEAbody-40K, ICE-20K is CD3-Her2 TMEAbody-20K, CD3-HER2-M is CD3-Her2 bispecific antibody comprising anti-CD3 antibody mutant, and CD3-Her2-WT is the wild-type CD3-Her2 bispecific antibody.

Specific dose design is as follows:

**Table 5 Specific dose design in the experiment**

| **Group number** | **Group** | **Test sample/contr ol sample** | **Date of administr ation** | **Dosage of administrati on (µg/kg)** | **Concentra tion of administra tion (µg/mL)** | **Capacity of administr ation (mL/kg)** |
|---|---|---|---|---|---|---|
| 1 | Low dose group of test sample | CD3-Her2-40 K | D1 | 3 | 1.2 | 2.5 |
| | | CD3-Her2-20 K | D15 | 3 | 1.2 | |
| | | | D46 | 300 | 120 | |
| 2 | High dose group of test sample | CD3-Her2-40 K | D1 | 80 | 32 | 2.5 |
| | | | D15 | 200 | 80 | |
| | | CD3-Her2-M | D46 | 80 | 32 | |
| 3 | Positive control group | CD3-Her2-W T | D1 | 3 | 1.2 | 2.5 |
| | | | D15 | 80 | 32 | |
| | | | D29 | - | - | |

| | | | | | | |
|---|---|---|---|---|---|---|
| Note: "-" represents that the animal has died. | | | | | | |

During the experiment, animals in the positive control group died approximately 10 hours after the administration of 80 µg/kg CD3-Her2-WT at D15. LYMPH and MONO decreased and NEUT increased at 3 hours and/or 10 hours after the administration of both 3 µg/kg and 80 µg/kg CD3-Her2-WT, and WBC decreased significantly at 3 hours after the administration of 80 µg/kg CD3-Her2-wt.

The increase of PT was observed at 10 hours after D1 administration; the increase of ALT, AST, CK, TG, GGT, BUN, CREA, K, P, AMY and LIP, and the decreased of GLU were observed before death.

The decrease of number and/or percentage of CD45⁺CD4⁺, CD45⁺CD8⁺, CD45⁺CD4⁺/CD45⁺CD8⁺, CD45⁺Ki-67⁺, CD45⁺CD4⁺Ki-67⁺, and CD45⁺CD8⁺Ki-67⁺cells were observed at 3 hours after administration of 80 µg/kg CD3-Her2-WT on D15 and before death.

Multiple cytokines (IFN-γ: 3571 pg/mL, IL-17A: 1061 pg/mL, IL-2: 556 pg/mL, IL-6: >2100 pg/mL, etc.) were significantly increased at 3 hours and 8 hours after administration of 80 µg/kg CD3-Her2-WT on D15. Gross autopsy showed no abnormality, and histopathological examination showed mild pulmonary cellulose exudation.

In summary, it is suggested that the death of the animal may be related to the cytokine release syndrome after administration.

The increase of NEUT and the decrease of LYMPH were observed in animals at 3 hours after each administration of the low-dose test sample group and high-dose test sample group. The increase of WBC was also observed at 3 hours after each administration of the low dose group, and no significant toxicological changes were observed at other time points.

In summary, under the condition of this experiment, rhesus monkey were intravenously infused with CD3-Her2-40K at doses of 3, 80, and 200 µg/kg, CD3-Her2-20K at doses of 3 and 300 µg/kg,CD3-Her2-M at a dose of 80 µg/kg, and/or CD3-Her2-WT at doses of 3 and 80 µg/kg.

Wherein, the maximum tolerance dose (MTD) of CD3-Her2-40K was greater than 200 µg/kg,the MTD of CD3-Her2-20K was greater than 300 µg/kg, the MTD of CD3-Her2-M was greater than 80 µg/kg,and the MTD of CD3-Her2-WT was less than 80 µg/kg.

Therefore, the above results indicate that CD3-Her2 TMEAbody-40K and CD3-Her2 TMEAbody-20K greatly increased the dosage of CD3 antibody administration (about 60-100 times higher than that of CD3-Her2-WT), significantly reducing cytokine storm and reducing toxic side effects.

### Discussion

Bispecific antibody activated by the tumor microenvironment in prior art is usually prepared by fusion expression of a substrate that can be recognized and cleaved and a blocker with a bispecific antibody. The substrate that can be recognized and cleaved and the blocker can be linked to the N terminus or C terminus of the bispecific antibody by a flexible linker (such as (G4S)n).

In the tumor microenvironment activated antibody in the present invention, by screening different CDR mutation sites in the variable region of the antibody, the substrate which is highly expressed in tumor and can be recognized and cleaved, and the blocker are chemically coupled to the CDR mutation site in the bispecific antibody. Thus, the antibody activity is blocked in normal tissues and activated in tumor environment.

All literatures mentioned in the present invention are incorporated herein by reference, as though each one is individually incorporated by reference. In addition, it should be understood that, after reading the above teachings of the present invention, those skilled in the art can make various changes or modifications to the present invention, these equivalents also fall within the scope as defined in the appended claims of the present application.

## Claims

1. A mutant CD3 antibody, wherein one or more mutations are introduced in the complementary determining regions (CDR regions) of the heavy chain variable region and/or light chain variable region of the mutant CD3 antibody relative to a wild-type CD3 antibody sequence, wherein the mutation is selected from the group consisting of:
a mutation where one or more amino acids are replaced with cysteine (Cys) at the position of M34, S53, Y61, D68, F104, W111, or F112 in the heavy chain variable region; and/or at the position of N54, K55, R56, W93, Y94 or N96 in the light chain variable region;
wherein, the affinity of the wild-type CD3 antibody for binding to CD3 is E0, the affinity of the mutant CD3 antibody for binding to CD3 is E1, and E1/E0 ≤ 5.5 (preferably E1/E0 ≤ 3, preferably ≤ 1.5, more preferably E1/E0 ≤ 1);
wherein the heavy chain variable region of the wild-type CD3 antibody comprises the following three complementary determining regions (CDRs):
VH-CDR1 as shown in SEQ ID NO.1,
VH-CDR2 as shown in SEQ ID NO.2, and
VH-CDR3 as shown in SEQ ID NO.3; and
the light chain variable region of the wild-type CD3 antibody comprises the following three complementary determining regions (CDRs):
VL-CDR1 as shown in SEQ ID NO.5,
VL-CDR2 as shown in SEQ ID NO.6, and
VL-CDR3 as shown in SEQ ID NO.7.

2. The mutant CD3 antibody of claim 1, wherein the mutant CD3 antibody comprises a mutation selected from the following group in the complementary determining regions of the heavy chain variable region corresponding to the wild-type CD3 antibody: M34C, S53C, Y61C, D68C, F104C, W111C, F112C, and a combination thereof, preferably a mutation of Y61C; and/or
comprises a mutation selected from the following group in the complementarity determining regions of the light chain variable region corresponding to the wild-type CD3 antibody: Y34C, N54C, K55C, R56C, W93C, Y94C, N96C, and a combination thereof, preferably a mutation of Y34C.

3. The mutant CD3 antibody of claim 1, wherein the mutant CD3 antibody comprises a modified mutant CD3 antibody having the following structure:
Ab-(P-R1)n
wherein,
Ab is a mutant CD3 antibody;
R1 is each independently a blocker;
P is a cleavable linker peptide;
n is a positive integer from 1 to 5;
"-" is a chemical bond or connector or linker.

4. The mutant CD3 antibody of claim 3, wherein R4 in P is site-specifically connected to an amino acid site of the mutant CD3 antibody selected from the group consisting of: M34, S53, Y61, D68, F104, W111, or F112 of the complementary determining region of the heavy chain variable region corresponding to the wild-type CD3 antibody; and/or Y34, N54, K55, R56, W93, Y94, or N96 of the complementarity determining region of the light chain variable region corresponding to the wild-type CD3 antibody.

5. A multispecific antibody, wherein the multispecific antibody comprises: the mutant CD3 antibody of any one of claims 1 to 4.

6. The multispecific antibody of claims 5, wherein the multispecific antibody further comprises one or more antigen binding regions targeting a target selected from the group consisting of: Her2, DLL3, CLDN6, EGFR, TGFβ, BCMA, B7H6, GUCY2C, CD38, CD123, CD19, CD20, CD22, B7-H3, GPC3, CD73, PMSA, CD28, 4-1BB, OX40, CD40, CD27, CTLA 4, PD1, PDL1, BCMA, GLP-1, Trop2, TIGIT, LAG-3, FGL1, TLR7.

7. A recombinant protein, wherein the recombinant protein comprises:
(1) the mutant CD3 antibody or the modified mutant CD3 antibody of any one of claims 1 to 4, or the multispecific antibody of claim 5 or claim 6;
(2) an optional polypeptide molecule or fragment with therapeutic function; and/or
(3) an optional protein functional domain that assists enhancing molecular physicochemical properties and pharmaceutical properties.

8. The recombinant protein of claim 7, wherein the polypeptide molecule or fragment with therapeutic function includes but is not limited to: a peptide molecule or fragment targeting a target selected from the group consisting of: Her2, DLL3, CLDN6, EGFR, TGFβ, BCMA, B7H6, GUCY2C, CD38, CD123, CD19, CD20, CD22, B7-H3, GPC3, CD73, PMSA, CD28, 4-1BB, OX40, CD40, CD27, CTLA 4, PD1, PDL1, BCMA, GLP-1, Trop2, TIGIT, LAG-3, FGL1, TLR7.

9. An isolated polynucleotide encoding the mutant CD3 antibody of any one of claims 1 to 4, or the multispecific antibody of claim 5 or claim 6, or the recombinant protein of claim 7 or claim 8.

10. A vector comprising the polynucleotide of claim 9.

11. A genetically engineered host cell comprising the vector of claim 10, or having the polynucleotide of claim 9 integrated into its genome.

12. A method for preparing the multispecific antibody of claim 5 or claim 6, wherein the method comprises the steps of:
(i) culturing the host cell of claim 11 under a suitable condition, thereby obtaining a mixture comprising the multispecific antibody of claim 5 or claim 6; and
(ii) purification and/or separation of the mixture obtained in step (i), thereby obtaining the multispecific antibody of claim 5 or claim 6.

13. A pharmaceutical composition comprising:
(I) the mutant CD3 antibody of any one of claims 1 to 4, the multispecific antibody of claim 5 or claim 6, or the recombinant protein of claim 7 or claim 8; and
(II) a pharmaceutically acceptable carrier.

14. An immunoconjugate, wherein the immunoconjugate comprises:
(a) the mutant CD3 antibody of any one of claims 1 to 4, the multispecific antibody of claim 5 or claim 6, or the recombinant protein of claim 7 or claim 8; and
(b) a coupling moiety selected from the group consisting of: a detectable label, a drug, a toxin, a cytokine, a radionuclide, an enzyme, and a combination thereof.

15. Use of the mutant CD3 antibody of any one of claims 1 to 4, the multispecific antibody of claim 5 or claim 6, the recombinant protein of claim 7 or claim 8, or the immunoconjugate of claim 14 for preparing (a) a detection reagent or kit; and/or (b) preparing a pharmaceutical composition for preventing and/or treating a cancer, tumor, or autoimmune disease.
